# EUROPEAN PATENT APPLICATION

(11) **EP 4 248 937 A1**
(43) Date of publication of application: **27.09.2023**
(21) Application number: 21911474.1
(22) Date of filing: 21.12.2021
(51) Int. Cl.: A61K 6/25

(54) **PLAQUE DISCLOSING COMPOSITION**

(30) Priority: 21.12.2020 KR 20200179473
(71) Applicant: Aiobio Co., Ltd., Seoul 06241 (KR); Tecozyme Inc, Seoul 03080 (KR)
(72) Inventor: YOON, Hong Cheol, Seoul 06583 (KR); NAM, Yoon, Seoul 04147 (KR)
(74) Representative: Jung, Minkyu
(86) International application number: PCT/KR2021/019503
(87) International publication number: WO 2022/139407

(57) **Abstract**

The present invention relates to a plaque disclosing composition, and a plaque disclosing agent comprising same. The plaque is expected to be formed, that is, the acquired pellicle. The present invention can variously acquire information about the amount and characteristics of dental plaque by analyzing the color and intensity of fluorescence emitted by riboflavin 5-phosphate sodium, and thus, in particular, is expected to be effectively used for observing early dental plaque.

## Description

### Technical Field

The present invention relates to a plaque disclosing composition, and specifically to a plaque disclosing composition containing riboflavin 5-phosphate sodium.

### Background Art

In general, a large number of microorganisms enter the human mouth via food, and since the internal conditions of the mouth are hot and humid, they provide a suitable place for the growth of microorganisms.

Microorganisms present in the mouth break down the carbohydrates in sugar and starch contained in food and thereby destroy the tissues surrounding the teeth. These microorganisms continuously attach to the tooth surfaces and thereby form a colorless film, known as a microbial film, on the tooth surface.

Such a microbial film on the tooth surface becomes larger due to the growth of new microorganisms and existing microorganisms and the accumulation of metabolites of the microorganisms and the host, which ultimately becomes the main cause of tooth decay, periodontal disease, halitosis, and gum disease. The microbial film on the tooth surface is not removed by a self-cleaning action in the oral cavity (i.e., saliva) but can only be removed by physical methods such as tooth brushing. If tooth brushing is neglected, microorganisms cannot be completely removed but will remain between the gums and teeth or between teeth, and as time passes, these microorganisms can attach to the teeth thereby formation of a microbial film again.

Meanwhile the microbial film on the tooth surfaces may be formed from a thin film known as a thin dental surface film or acquired pellicle on the tooth surface. Currently, a method for confirming a microbial film on tooth surfaces using an optical device has been developed, but the conventional technology has a problem in that it is impossible to observe a microbial film on the tooth surfaces at the initial stage in which the film has just been formed or is expected to be formed. Additionally, in the case of the method of confirming the presence of a microbial film on the tooth surfaces using a chemical coloring product, aesthetics may be poor due to residual substances remaining after use, and there may be an inconvenience that the residual material after use reduces aesthetics and the microbial film on the tooth surfaces, to which the chemical coloring product is directly adsorbed, should be physically scraped off.

### Detailed Description of the Invention

### Technical Problem

The present inventors have extensively studied and made efforts to develop a plaque disclosing composition capable of observing the expected site of formation of a microbial film on the tooth surfaces and the initial microbial film on the tooth surfaces. As a result, they have confirmed that riboflavin 5-phosphate sodium (RPS) can be colored not only in a biofilm but also in acquired pellicle, thereby completing the present disclosure.

Accordingly, an object of the present disclosure is to provide a plaque disclosing composition containing riboflavin 5-phosphate sodium.

Another object of the present disclosure is to provide a disclosing solution containing a plaque disclosing composition that contains riboflavin 5-phosphate sodium.

### Technical Solution

The present inventors have extensively studied and made efforts to develop a plaque disclosing composition capable of observing the expected site of formation of a microbial film on the tooth surfaces and the initial microbial film on the tooth surfaces. As a result, they have confirmed that riboflavin 5-phosphate sodium (RPS) can be colored not only in a biofilm but also in an acquired pellicle.

The present disclosure relates to a disclosing solution which contains a plaque disclosing composition containing riboflavin 5-phosphate sodium.

Hereinafter, the present disclosure will be described in more detail.

An aspect of the present disclosure relates to a plaque disclosing composition containing riboflavin 5-phosphate sodium.

In the present disclosure, "riboflavin 5-phosphate sodium" is represented by the following Formula (1), has a molecular formula of C₁₇H₂₄N₄NaO₁₁P, a molecular weight of 514.4, and refers to the sodium phosphate form of riboflavin, which is a major growth-promoting factor of naturally-occurring vitamin B complex.

Specific examples of a salt of the riboflavin 5-phosphate sodium may include riboflavin 5-phosphate sodium salt dihydrate and riboflavin 5-phosphate sodium salt hydrate, but are not limited thereto.

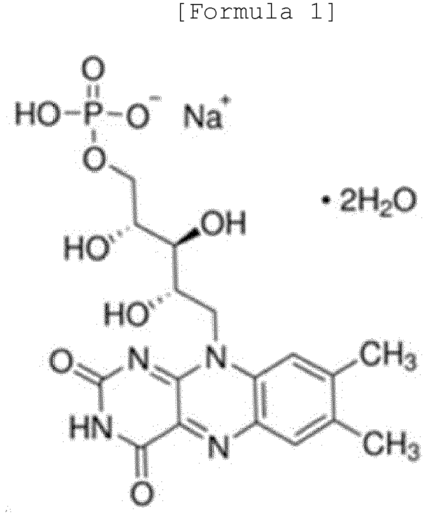

The composition may be one which colors (stains) a microbial film on the tooth surfaces or acquired pellicle.

In the present disclosure, "a microbial film on the tooth surfaces," also known as dental plaque, is an initial material for forming dental calculus, which becomes the main cause of tooth decay and periodontal disease, and refers to a state in which bacteria adhere to the acquired pellicle and form colonies.

In the present disclosure, "acquired pellicle" refers to an acquired thin membrane, which is a non-structural, acellular organic film formed on the surfaces of teeth, prostheses, dental calculus, artificial objects, *etc*., and is reformed within a few minutes even after brushing, and while it serves to protect teeth from acids, it also plays an important role in helping bacteria attach to the teeth.

The method for obtaining riboflavin 5-phosphate sodium represented by Formula 1 of the present disclosure is not particularly limited, and it may be chemically synthesized using a method known in the art or a commercially available material may be used.

The riboflavin 5-phosphate sodium may exist in unsolvated as well as solvated forms. Riboflavin 5-phosphate sodium of the present disclosure may exist in either crystalline or amorphous form, and all such physical forms are within the scope of the present disclosure.

The riboflavin 5-phosphate sodium generates negatively-charged riboflavin phosphate in an aqueous solution, and this material can be electrostatically bound to the microbial film on the tooth surfaces and acquired pellicle.

At this time, since riboflavin phosphate emits fluorescence of light green to red light depending on the concentration when irradiated with light at 405 nm, when irradiated at 405 nm after treatment with the composition of the present disclosure, the area to be formed and the initial microbial film on the tooth surfaces can be observed.

The concentration of riboflavin 5-phosphate sodium may be in the range of 0.1 mg/mL to 50.0 mg/mL, 0.1 mg/mL to 40.0 mg/mL, 0.1 mg/mL to 30.0 mg/mL, 0.1 mg/mL to 20.0 mg/mL, 0.1 mg/mL to 10.0 mg/mL, 0.1 mg/mL to 5.0 mg/mL, 0.1 mg/mL to 4.0 mg/mL, 0.1 mg/mL to 3.0 mg/mL, 0.1 mg/mL to 2.0 mg/mL, or 0.1 mg/mL to 1.0 mg/mL. When the concentration of the riboflavin 5-phosphate sodium is less than 0.1 mg/mL, fluorescence intensity may be too low, and thus a microbial film on the tooth surfaces may not be observed. Additionally, when the concentration of riboflavin 5-phosphate sodium exceeds 50.0 mg/mL, a phenomenon in which the intensity of fluorescence is rather decreased due to the quenching effect of the fluorescent material and aesthetics may be damaged due to the residual material.

Meanwhile, since the riboflavin 5-phosphate sodium of the present disclosure is a food additive and water-soluble vitamin (no dose limitations) certified by the Ministry of Food and Drug Safety of Korea, it has an advantage in that it is safe to use as a composition for coloring tooth surfaces, which is an oral composition.

Additionally, as mentioned above, the riboflavin 5-phosphate sodium of the present disclosure has advantages in that it is easy to use because it emits fluorescence only when irradiated with light at 405 nm and does not require separate washing, compared to conventional compositions for coloring tooth surfaces, in which a fluorescent chemical coloring product is applied to the tooth surface and a separate washing process is essentially accompanied thereafter, and that it is aesthetically excellent.

The plaque disclosing composition of the present disclosure is not particularly limited, but may be in the form of powder, granule, tablet, emulsion, aerosol, soft or hard capsule, sterile injection solution, or sterile powder, and may additionally contain a dispersant or stabilizer.

The plaque disclosing composition of the present disclosure may contain commonly used polishing agents, wetting agents, foaming agents, binders, sweeteners, pH adjusting agents, preservatives, pharmacological agents, fragrances, brightening agents, pigments, solvents, etc., depending on the formulation and purpose of use.

An aspect of the present disclosure relates to a disclosing solution, which contains a plaque disclosing composition that contains riboflavin 5-phosphate sodium.

In the present disclosure, the disclosing solution refers to a preparation which is used for staining or coloring a microbial film on the tooth surfaces (dental plaque), acquired pellicle, *etc*.

The formulation type of the disclosing solution is not particularly limited, but may be in the form of a liquid, gel, cream, film, patch, powder, tablet, or solid preparation.

The descriptions which are common between the composition for coloring tooth surfaces and the tooth coloring agent of the present disclosure are omitted in order to avoid excessive complexity in the present specification.

### Advantageous effects

The present disclosure relates to a plaque disclosing composition and a disclosing solution including the same, in which the plaque disclosing composition according to the present disclosure can be colored (stained) not only for a microbial film on the tooth surface (biofilm) but also for the expected site of formation of a microbial film on the tooth surfaces (*i.e*., acquired pellicle), and it is characterized that various pieces of information on the amount and properties of the microbial film on the tooth surface can be obtained by analyzing the color and intensity of fluorescence emitted by riboflavin 5-phosphate sodium of the present disclosure; therefore, it is expected to be effectively used, especially for the purpose of observing the initial microbial film on the tooth surface.

### Brief description of the drawings

FIG. 1 illustrates the evaluation results of the fluorescence intensity of a composition according to an embodiment of the present disclosure, in which FIG. 1a shows images photographed using a Qraycam Pro (AIOBIO, Seoul, Korea) (left: white light, right: blue light) and FIG. 1b is a graph illustrating the results measured using a fluorescence spectrometer.
FIG. 2 illustrates the evaluation results of the staining of the acquired pellicle of the composition according to an embodiment of the present disclosure (left: white light, right: blue light).
FIG. 3 illustrates the evaluation results of staining of the composition according to an embodiment of the present disclosure on a *Streptococcus mutans* biofilm (left semi-circle: control group, right semi-circle: experimental group) .
FIG. 4 illustrates the evaluation results of staining of the composition according to an embodiment of the present disclosure on a polymicrobial biofilm (left semi-circle: control group, right semi-circle: experimental group)

### Mode for the invention

Hereinafter, the present disclosure will be described in more detail through examples. These examples are only for explaining the present disclosure in more detail, and it will be apparent to those skilled in the art that the scope of the present disclosure is not limited by these examples according to the gist of the present disclosure.

### Experimental Example 1. Evaluation of fluorescence intensity of riboflavin 5-phosphate sodium (RPS)

Sterile distilled water (DW, (1)), gargle (GG, Soom Mouthwash, (2)), saliva (S(F-X), (3)) and filtered saliva (S(F-O), (4)) 100 mg/mL RPS stock solution (plates were prepared after preparation by diluting at concentrations of 100 mg/mL, 50 mg/mL, 25 mg/mL, 12.5 mg/mL, 6.25 mg/mL, 3.12 mg/mL, 1.5 mg/mL, 0.78 mg/mL, 0.39 mg/mL, 0.19 mg/mL, and 0.1 mg/mL.

Each plate prepared was photographed with the Qraycam Pro (AIOBIO, Seoul, Korea) to obtain white light and blue light images. Then, fluorescence intensity was measured using a fluorescence spectrometer (Varioskan, excitation = 405 nm/emission = 520 nm).

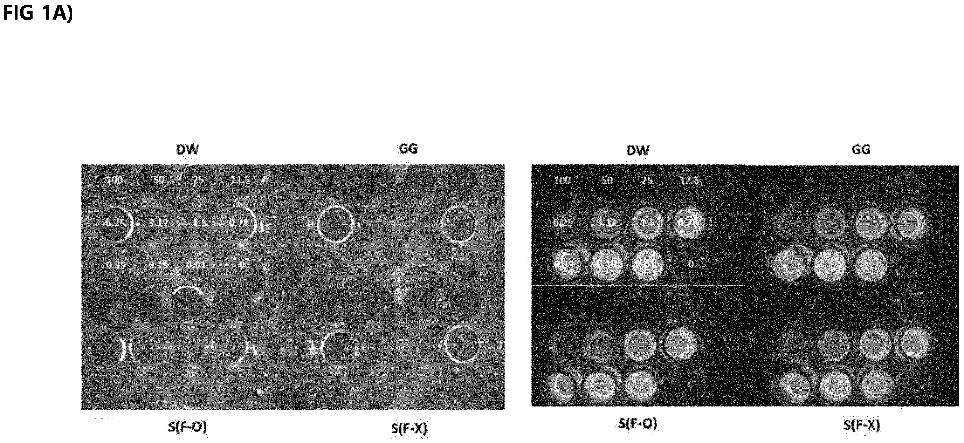

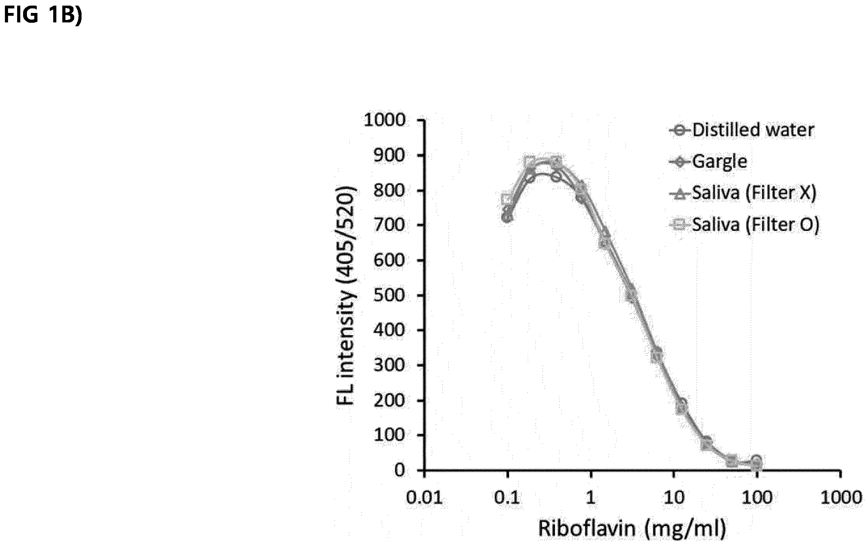

As can be seen in FIG. 1, as a result of visual evaluation of the images, no difference in fluorescence was observed between solvents in both white light (left of FIG. 1a) and blue light (right of FIG. 1a), it was found that as with a shift in the solvent concentration from a high concentration (100 mg/mL) to a low concentration (0.01 mg/mL), red fluorescence was converted to green fluorescence.

Additionally, as a result of measuring the fluorescence intensity of RPS for each concentration using a fluorescence spectrometer, it was found that there was a trend in which the fluorescence intensity was increased from 0.01 mg/mL to 0.39 mg/mL, and then decreased again. There was no difference with regard to the increase-decrease trend depending on the solvent, and the maximum fluorescence intensity was confirmed to be a concentration of 0.4 mg/mL in all solvents (FIG. 1b).

### Experimental Example 2. Evaluation of RPS on staining acquired pellicle (AP)

In order to evaluate whether RPS also stains the acquired pellicle, which is a component of saliva, the experiment was performed as follows.

An artificial coating was formed by immersing a bovine teeth specimen in artificial saliva for 4 hours. Specimens with acquired pellicle were immersed in 2-tone (2T), RPS50 (RPS; 50 mg/mL), and RPS0.4 (RPS; 0.4 mg/mL). After the immersion, water washing was performed with distilled water three times. Then, immediately after the application of a staining agent, the images were photographed using the Qraycam Pro after the 1st washing, the 2nd washing, and the 3rd washing, and the degree of staining per each step was confirmed.

A plaque staining agent, 2-tone (2T), used in clinical practice was diluted to a concentration of 10% and set as a control group. As an experimental group, two concentrations, 50 mg/mL (the highest concentration at which RPS was completely dissolved) and 0.4 mg/mL (the strongest fluorescence intensity), were selected based on the results of Experimental Example 1 above.].

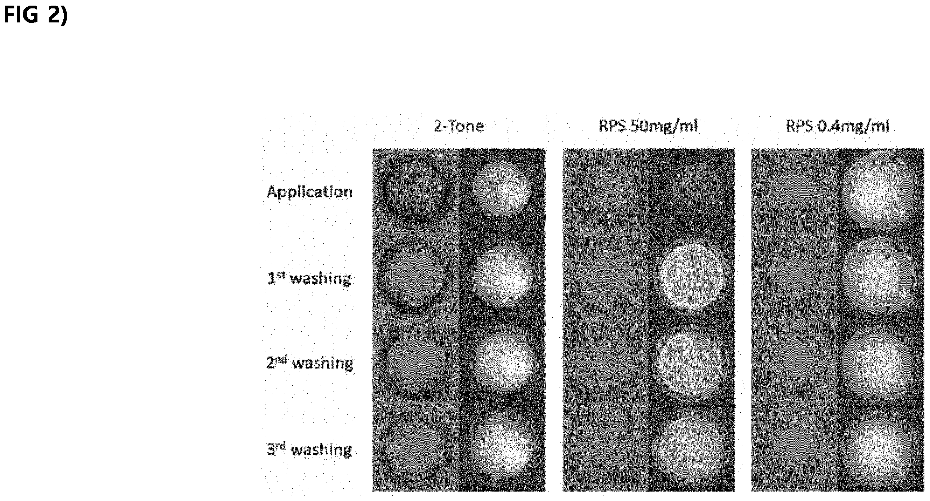

As can be seen in FIG. 2, immediately after the application of a staining agent, both RPS50 and RPS0.4 were stained in the acquired pellicle. After the third water washing, as for RPS50, fluorescence was observed only in blue light (right side of FIG. 2), and as for RPS0.4, fluorescence was lost after water washing. In particular, as for RPS50, there was a characteristic change in the fluorescence before water washing from red to yellow after.

These results indicate that each fluorescence becomes different depending on the residual amount of RPS attached to the microbial film on the tooth surfaces; therefore, it is possible to obtain more diverse information on the amount and properties of the microbial film on the tooth surfaces by analyzing the color and intensity of the fluorescence emitted by RPS.

### Experimental Example 3. Evaluation of staining of RPS on Streptococcus mutans

Since *S. mutans* is a major pathogen in the oral cavity and forms biofilms well, the following experiment was performed to evaluate the degree of staining by RPS on biofilms using a single strain *S. mutans* biofilm model.

After the formation of acquired pellicle on bovine tooth specimens, *S. mutans* seeds were inoculated. In the same manner as in Experimental Example 2, the specimens were divided into three groups (2T, RPS50, and RPS0.4) and the biofilms were matured for 24, 48, and 72 hours per group. Thereafter, Qraycam Pro images were photographed immediately after applying the staining agent to the biofilm at each time (24, 48, and 72 hours) (no washing) and after the third water washing (after washing three times). At this time, the image obtained from the acquired pellicle experiment of Experimental Example 2 was used as a control image.

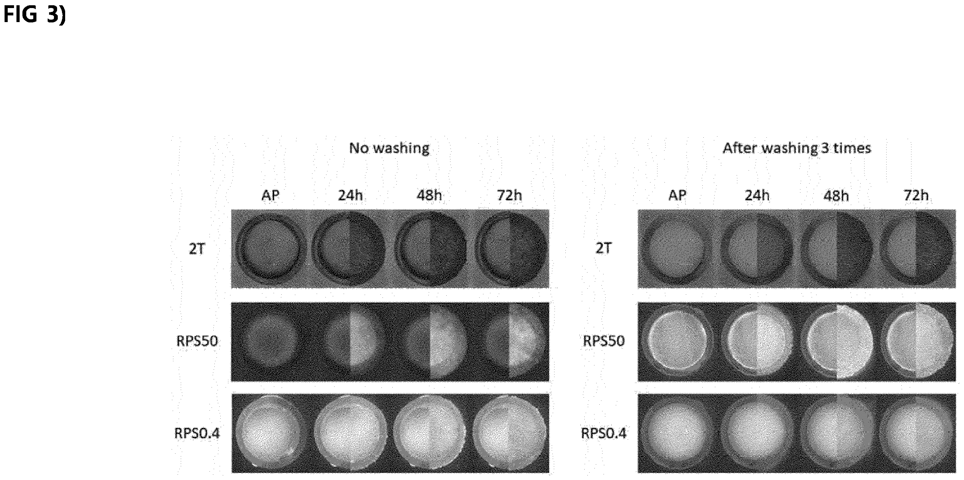

As can be seen in FIG. 3, immediately after the application of the staining agent, RPS50 showed red fluorescence spread throughout the specimen, and after the third water washing, RPS50 exhibited a characteristic in which the yellow fluorescence changed to (yellow + red) fluorescence as the incubation time increased.

Additionally, immediately after the application of the staining agent, only yellow fluorescence was weakly observed in the 24-hour biofilm of RPS0.4, and red fluorescence was observed in the center of the specimen in the 48-hour and 72-hour biofilms. After the third water washing, almost no fluorescence was observed in the 24-hour biofilm, and weak yellow fluorescence was observed in the 48-hour biofilm, and slight red fluorescence was observed in the 72-hour biofilm.

These results indicate that since fluorescence of each different color is displayed depending on the residual amount of RPS attached to the microbial film on the tooth surfaces and the degree of maturity of the microbial film on the tooth surfaces, more diverse information can be obtained with regard to the color and intensity of the fluorescence emitted by the RPS by analyzing the maturity, amount and nature of the microbial film on the tooth surfaces.

### Experimental Example 4. Evaluation of staining of RPS on microcosm biofilm

Since a microbial film on the tooth surfaces in the oral cavity is a polymicrobial population, the following experiment was performed to evaluate the degree of staining of RPS on biofilms using a multi-species microcosm biofilm model which simulates the polymicrobial population.

A bovine tooth specimen was inoculated with stimulating saliva to form a polymicrobial biofilm. In the same manner as in Experimental Example 2, the specimens were divided into three groups (2T, RPS50, and RPS0.4) and a staining agent was applied to biofilms formed for 24, 48, and 72 hours per group. Thereafter, the images were photographed using the Qraycam Pro immediately after applying the staining agent to the biofilms for each time point (24, 48, and 72 hours) (no washing) and after the third water washing. At this time, the image obtained from the acquired pellicle experiment of Experimental Example 2 was used as a control image.

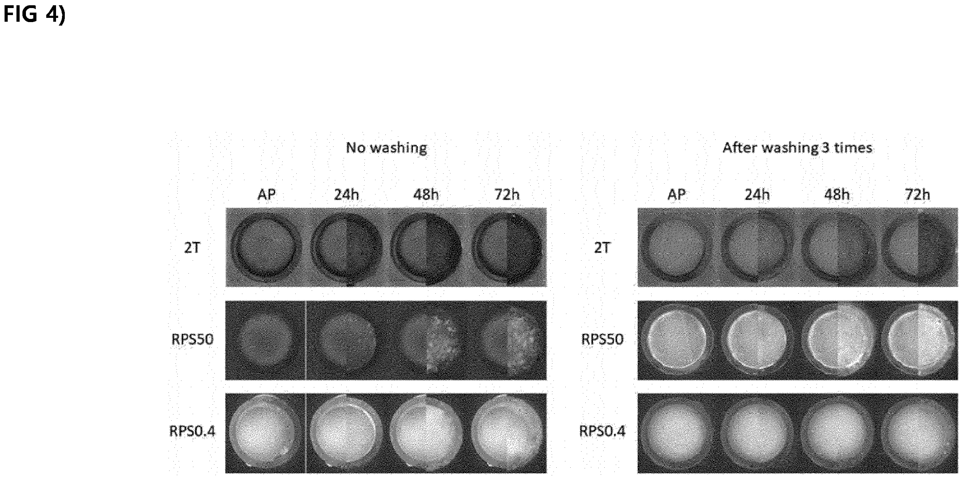

As can be seen in FIG. 4, compared to the results of *S. mutans* biofilm of Experimental Example 3, there was no characteristic difference. However, the overall amount of biofilm was observed to be small, which may be interpreted as a result of inhibiting the formation of a microbial film on the tooth surfaces due to the interaction and competition among bacteria in the case of a biofilm of a polymicrobial population.

### Subconclusion

The plaque disclosing composition according to the present disclosure can be colored (stained) not only for the microbial film on the tooth surfaces (biofilm) but also for the expected site of formation of a microbial film on the tooth surfaces (*i.e*., acquired pellicle), and it is characterized that various information on the amount and properties of the microbial film on the tooth surface can be obtained by analyzing the color and intensity of fluorescence emitted by riboflavin 5-phosphate sodium of the present disclosure; therefore, it is expected to be effectively used especially for the purpose of observing the initial microbial film on the tooth surfaces.

## Claims

1. A plaque disclosing composition comprising riboflavin 5-phosphate sodium.

2. The composition of claim 1, wherein the composition colors a microbial film on the tooth surfaces or acquired pellicle.

3. The composition of claim 1, wherein the riboflavin 5-phosphate sodium is contained at a concentration of 0.1 mg/mL to 50.0 mg/mL.

4. The composition of claim 1, wherein the composition is formulated into powders, emulsions, aerosols, or soft or hard capsules.

5. The composition of claim 1, wherein the composition emits fluorescence when irradiated with light at 405 nm.

6. The composition of claim 1, wherein the composition does not require separate water washing after coloring the tooth surface.

7. A disclosing solution comprising the composition according to any one of claims 1 to 6.

8. The solution of claim 7, wherein the disclosing solution is formulated into liquids, gels, creams, powders, films, patches, tablets, or solids.

9. The solution of claim 7, wherein the disclosing solution does not require separate water washing after disclosing the tooth surface.
